Europäisches Patentamt

European Patent Office

Office européen des brevets

(11) Numéro de publication: **0 378 956**
**A1**

(12) **DEMANDE DE BREVET EUROPEEN**

(21) Numéro de dépôt: 89430033.4

(22) Date de dépôt: 23.11.89

(51) Int. Cl.5: **A61K 7/16, A61K 7/00**

Le titre de l'invention a été modifié (Directives relatives à l'examen pratiqué à l'OEB, A-III, 7.3)

(30) Priorité: **25.11.88 ES 8803612**

(43) Date de publication de la demande:
**25.07.90 Bulletin 90/30**

(84) Etats contractants désignés:
**BE CH DE FR GB IT LI**

(71) Demandeur: **MAGRINA, Josep Maria**
**23, Avenida Coll del Portell**
**E-08024 Barcelona(ES)**

(72) Inventeur: **MAGRINA, Josep Maria**
**23, Avenida Coll del Portell**
**E-08024 Barcelona(ES)**

(74) Mandataire: **Marek, Pierre**
**28 & 32 rue de la Loge**
**F-13002 Marseille(FR)**

(54) **Produit pour l'hygiène et la prophylaxie dentaires.**

(57) Produit pour l'hygiène et la prophylaxie dentaires, caractérisé en ce qu'il consiste en un corps (1) dont le contour et la structure constituent un récipient fermé en forme de capsule qui, par sa configuration, sert d'enveloppe ou de revêtement à un produit (2) ayant une forme concordante avec celle du récipient et formant le noyau du corps, l'ensemble étant conçu de façon à ce que le corps enrobant (1) étant lui-même formé d'un produit fait pour être dissout par la salive et les sucs de la bouche, le contenu (2) soit constitué par un produit qui possède des caractéristiques et des propriétés détergentes et antiseptiques pour les parties intérieures de la bouche, et notamment pour les dents, de sorte qu'après dissolution du contenant ou enveloppe (1), le produit qui constitue le noyau (2) puisse être dissout et réparti dans la bouche dans les endroits à nettoyer sans qu'il soit nécessaire d'utiliser une brosse à dent ou un ustensile similaire.

Fig.1

EP 0 378 956 A1

L'invention concerne un nouveau produit pour l'hygiène et la prophylaxie dentaires.

Il est bien connu que dans le domaine de l'hygiène et de la propreté buccales, l'un des facteurs les plus importants à prendre en compte est le nettoyage et la désinfection des dents. Pour ce faire, depuis très longtemps, on a utilisé différents produits qui, lors de leur mise en oeuvre à l'aide d'une brosse à dent, permettent à l'utilisateur d'éliminer d'une façon très efficace les produits nocifs et les impuretés collées aux dents et logées dans les espaces interdentaires.

Bien que l'utilisation de la brosse à dent, sous toutes ses formes, ait été et continu à être largement répandue, on sait également qu'il n'est pas toujours possible de s'en servir, car on n'a pas l'habitude de l'emmener partout avec soi, de sorte qu'il n'est pas toujours possible d'effectuer un nettoyage buccal après chaque repas, comme cela serait souhaitable.

Pour remédier à cet inconvénient, on a mis au point un nouveau produit pour la prophylaxie et l'hygiène dentaires permettant le nettoyage des dents sans utilisation d'une brosse à dent classique ; autrement dit, l'utilisateur peut porter ce produit sur lui, ou le trouver dans n'importe quel local public, étant donné que sa taille réduite ainsi que son transport et son maintien à un niveau élevé de stérilisation, permettent d'écarter tout danger lors de son utilisation, quel que soit l'endroit où il est utilisé.

Le nouveau produit pour la prophylaxie et l'hygiène dentaires selon l'invention se caractérise par un corps généralement de forme sphérique, ou quasi sphérique, aplatie, ovoïde ou de forme similaire, dépourvue d'arête, dont le contour et la structure constituent un corps fermé en forme de récipient lequel, par sa configuration, sert d'enveloppe ou de revêtement à un produit qui se présente sous une forme concordante à celle du récipient et constitue le noyau du corps. L'ensemble corps/noyau est conçu expressément de façon à ce que le corps enrobant étant lui-même formé d'un produit fait pour être dissout par la salive et les sucs de la bouche, le contenu ou noyau soit un produit qui possède des caractéristiques et des propriétés détergentes et antiseptiques pour les parties intérieures de la bouche, et notamment, les dents, de sorte qu'après la dissolution du contenant ou enveloppe,le produit qui constitue le noyau puisse être dissout et réparti dans la bouche dans les endroits à nettoyer sans qu'il soit nécessaire d'utiliser une brosse à dent ou un ustensile similaire.

Une fois que le produit constituant le noyau a rempli sa fonction, celui-ci peut être avalé ou expulsé, de manière à ce que le nettoyage de la bouche soit achevé de façon adéquate et sans avoir recours à des éléments auxiliaires, c'est-à-dire, des brosses à dents ou des objets similaires, lesquels ne peuvent ou ne pourraient que contribuer au nettoyage.

La planche de dessins annexée au présent mémoire descriptif montre, à titre d'exemple non limitatif, une représentation du produit pour la prophylaxie et l'hygiène dentaires.

Dans ces dessins :
- la figure 1 est une vue en coupe ou section transversale du produit selon l'invention.
- la figure 2 est une vue partielle et en coupe selon la ligne II-II de la figure 1 ; et,
- la figure 3 est un mode de transport ou d'utilisation du produit.

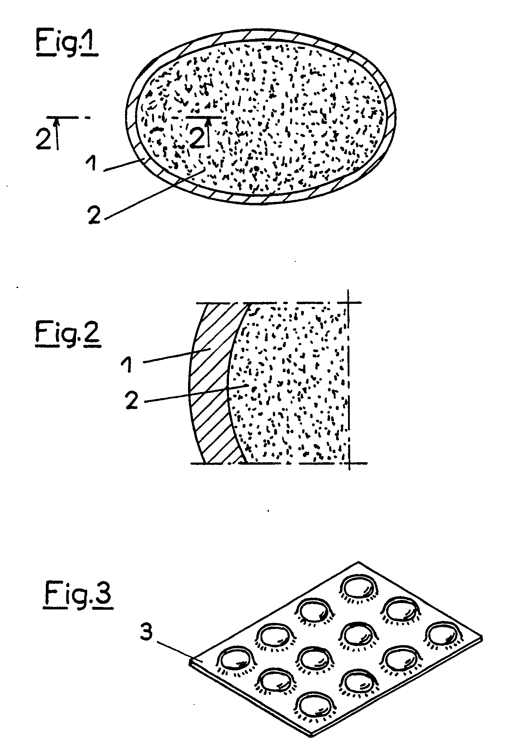

Selon les figures 1 et 2 et en se reportant aux références numériques portées sur ces dernières, on voit que le produit selon l'invention comprend un corps enrobant 1 ou enrobage lequel, ayant la forme d'une capsule fermée, constitue l'enveloppe d'un noyau 2 dont la forme concorde avec celle du récipient, ce noyau étant constitué par un produit 2 présentant des caractéristiques ou actions antiseptiques et détergentes pour l'intérieur de la bouche. Les corps qui constituent le produit selon l'invention peuvent être conditionnés dans des emballages individuels ou collectifs. La figure 3 représente un exemple d'emballage collectif 3 à usages multiples permettant d'apprécier les avantages qu'offre le transport de ce produit dont les qualités restent inchangées et qui garde, comme s'il s'agissait d'un produit pharmaceutique spécifique, un degré élevé de pureté, de stérilisation, et de conservation, lors de son transport et de son entretien.

Dans le but de fournir des informations complètes au sujet de la structure interne du nouveau produit pour la prophylaxie et l'hygiène dentaires, objet de la présente invention, on expose ci-après un mode de réalisation de l'invention, lequel est donné naturellement à titre d'exemple non limitatif, et selon lequel on obtient un produit qui répond en tous points aux exigences de l'objet qui est revendiqué.

Le corps 1 qui constitue le récipient fermé en forme de capsule, est constitué d'une gélatine durcie laquelle sert d'enveloppe ou de revêtement du produit actif qui forme le noyau dudit corps.

La composition du produit formant le noyau 2 peut être la suivante :

| Sels phosphatés ou calciques ..... | 1,50 g |
|---|---|
| Gomme arabique ..... | 0,03 g |
| Détergent neutre pulvérisé ..... | 0,20 g |
| Saccharine ..... | 0,01 g |
| Monofluorophosphate sodique ..... | 0,02 g |
| Excipient c.s.p ..... | 2,00 g |

Toutes ces substances sont suffisamment denses pour que grâce à l'action de la langue et des autres mouvements de la bouche, elles pénètrent, diluent et entraînent les restes de repas et de salive avec les détritus, avant qu'ils ne fermentent.

La principale qualité du produit est sa facilité d'emploi puisqu'il peut être porté sur soi de façon hygiénique pour être utilisé aisément après chaque repas, le tout étant équivalent à un rinçage de bouche.

**Revendications**

1. - Produit pour l'hygiène et la prophylaxie dentaires, caractérisé en ce qu'il consiste en un corps (1) dont le contour et la structure constituent un récipient fermé en forme de capsule qui, par sa configuration, sert d'enveloppe ou de revêtement à un produit (2) ayant une forme concordante avec celle du récipient et formant le noyau du corps, l'ensemble étant conçu de façon à ce que le corps enrobant (1) étant lui-même formé d'un produit fait pour être dissout par la salive et les sucs de la bouche, le contenu (2) soit constitué par un produit qui possède des caractéristiques et des propriétés détergentes et antiseptiques pour les parties intérieures de la bouche, et notamment pour les dents, de sorte qu'après dissolution du contenant ou enveloppe (1), le produit qui constitue le noyau (2) puisse être dissout et répartie dans la bouche dans les endroits à nettoyer sans qu'il soit nécessaire d'utiliser une brosse à dent ou un ustensile similaire.

2. - Produit selon la revendication 1, caractérisé en ce qu'il présente une forme ou contour dépourvue d'arête telle qu'une forme sphérique, ou quasi sphérique, ou aplatie, ou ovoïde, ou une forme similaire.

3. - Produit selon l'une des revendications 1 ou 2, caractérisé en ce que le corps enrobant ou enrobage (1) est constitué d'une gélatine durcie.

4. - Produit selon l'une quelconque des revendications 1 à 3, caractérisé en ce que le produit actif (2) a la composition suivante :

| Sels phosphatés ou calciques ..... | 1,50 g |
|---|---|
| Gomme arabique ..... | 0,03 g |
| Détergent neutre pulvérisé ..... | 0,20 g |
| Saccharine ..... | 0,01 g |
| Monofluorophosphate sodique ..... | 0,02 g |
| Excipient c.s.p ..... | 2,00 g |

Fig.1

2

1

2

2

Fig.2

1

2

Fig.3

3

# DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | US-A-4 292 304  (BARELS et al.)<br>* Colonne 2, lignes 9-50; colonne 3, ligne 46 - colonne 4, ligne 68; revendications *<br>--- | 1-4 | A 61 K    7/16<br>A 61 K    7/00 |
| X | PATENT ABSTRACTS OF JAPAN, vol. 10, no. 280 (C-374)[2336], 24 septembre 1986; & JP-A-61 100 516 (SHISEIDO)<br>* Résumé *<br>--- | 1-3 | |
| X,Y | FR-A-2 383 661  (BLENDAX-WERKE R. SCHNEIDER GmbH & CO.)<br>* En entier *<br>--- | 1-3 | |
| Y | FR-A-  791 179  (MESSNER)<br>* Page 1, lignes 29-34; page 2, ligne 98 - page 3, ligne 8; page 4, lignes 59-89; revendications *<br>--- | 1-3 | |
| X | FR-A-2 535 608  (DUBARRY et al.)<br>* En entier *<br>--- | 1-3 | **DOMAINES TECHNIQUES RECHERCHES (Int. Cl.5)** |
| X,P | WO-A-8 900 418  (YUASA)<br>* Résumé *<br>----- | 1-3 | A 61 K |

Le présent rapport a été établi pour toutes les revendications

| Lieu de la recherche | Date d'achèvement de la recherche | Examinateur |
|---|---|---|
| LA HAYE | 19-03-1990 | FISCHER J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

................................................................
& : membre de la même famille, document correspondant

EPO FORM 1503 03.82 (P0402)